(19) **Europäisches Patentamt / European Patent Office / Office européen des brevets**

(11) **EP 2 617 832 B1**

(12) **EUROPEAN PATENT SPECIFICATION**

(45) Date of publication and mention of the grant of the patent:
**13.05.2020 Bulletin 2020/20**

(21) Application number: **11825009.1**

(22) Date of filing: **05.09.2011**

(51) Int Cl.:
*C12P 1/00* (2006.01)   *C12P 7/56* (2006.01)
*C12P 7/40* (2006.01)   *B01D 65/06* (2006.01)
*B01D 65/02* (2006.01)   *B01D 61/18* (2006.01)
*B01D 63/02* (2006.01)   *B01D 71/34* (2006.01)
*C12M 1/26* (2006.01)   *C12M 1/00* (2006.01)

(86) International application number:
**PCT/JP2011/070109**

(87) International publication number:
**WO 2012/036003 (22.03.2012 Gazette 2012/12)**

(54) **PRODUCTION METHOD FOR CHEMICALS BY CONTINUOUS FERMENTATION**

HERSTELLUNGSVERFAHREN FÜR CHEMIKALIEN DURCH KONTINUIERLICHE FERMENTIERUNG

PROCÉDÉ DE PRODUCTION DE PRODUITS CHIMIQUES PAR FERMENTATION CONTINUE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **14.09.2010 JP 2010205239**

(43) Date of publication of application:
**24.07.2013 Bulletin 2013/30**

(73) Proprietor: **Toray Industries, Inc.**
**Tokyo 103-8666 (JP)**

(72) Inventors:
• **KOBAYASHI, Atsushi**
  **Shiga 520-8558 (JP)**
• **CHEON, Jihoon**
  **Shiga 520-8558 (JP)**
• **TAKEUCHI, Norihiro**
  **Shiga 520-8558 (JP)**
• **NISHIDA, Makoto**
  **Shiga 520-8558 (JP)**
• **TANAKA, Yuji**
  **Shiga 520-8558 (JP)**
• **MINEGISHI, Shin-ichi**
  **Chiba 279-8555 (JP)**

(74) Representative: **Hoefer & Partner Patentanwälte mbB**
**Pilgersheimer Straße 20**
**81543 München (DE)**

(56) References cited:
JP-A- 2000 317 273    JP-A- 2002 126 470
JP-A- 2008 099 667    JP-A- 2008 199 948
JP-A- 2008 289 959    JP-A- 2009 065 966
JP-A- 2009 065 970    JP-A- 2011 188 791
US-A1- 2009 269 812

EP 2 617 832 B1

**Description**

TECHNICAL FIELD

The present invention relates to a method for producing chemicals through continuous fermentation using a separation membrane.

BACKGROUND ART

Separation membranes have been used in a wide range of fields, including the field of water treatment such as drinking water production, water purification, and waste water treatment, the field of drug production, and the food industry, and also have application in the field of fermentation. In the field of water treatment such as drinking water production, water purification, and waste water treatment, separation membranes are used for the removal of water impurities as an alternative means of the conventional sand filtration and coagulation sedimentation. The waste water treatment and the food industry widely employ the separation membrane technique in which large quantities of high-concentration waste water or feedstock are charged into a processing vessel and treated while maintaining the microbes at high concentration, because the technique offers a high removal ratio and produces a high-purity liquid.

In such fields including the field of water treatment and the food industry using separation membranes, there is a need to improve the permeation performance for cost reduction. To this end, for example, there have been attempts to reduce the membrane area with the use of a separation membrane that excels in permeation performance, and to downsize the device, thereby reducing the facility cost, the membrane replacement cost, and the installation area. For cost considerations, a hollow fiber membrane having a wide filter area per installation area has attracted interest.

In recent years, continuous production methods that involve culturing of microbes and cultured cells have been actively proposed. In this technique, microbes or cultured cells are filtered through a separation membrane, and the separated microbes or cultured cells are retained or refluxed in the culture medium simultaneously when collecting the product from the filtrate. In this way, the technique can maintain high microbe or cell concentrations in the culture medium. For example, Patent Document 1 proposes increasing productivity by improving microbe or cell concentrations in a culture medium and maintaining high concentrations in a continuous fermentation performed with a separation membrane.

Despite the active studies of the separation membrane technique for microbe culture and microbes separation, the treatment of a biological liquid to be treated such as a culture medium through membrane separation involves serious fouling of the membrane by the microbes, sugar, protein, lipid, or the like contained in the liquid to be treated. This is problematic because it quickly deteriorates the membrane permeation flux.

Another problem of the separation membrane technique for microbe culture and microbes separation is the control of microbe proliferation. The membrane separation using the membrane filter can maintain high microbe or cell concentrations, and can thus produce products at high production rates with large numbers of microbes. However, increasing the fermentation time to take advantage of the continuous fermentation allows the microbes to continuously proliferate, and excessively increases the microbe concentration, thereby increasing the transmembrane pressure. One possible solution is to draw out some of the microbes during the operation and control the microbe concentration. However, this may cause a problem in the treatment of the withdrawn microbes, or may increase the possibility of contamination with the other microbes in the fermentor during or after the microbes are drawn out.

In common membrane separation processes, a membrane surface is washed on a regular or irregular basis to remove the materials adhered or adsorbed to the membrane surface (for example, SS (Suspended Solid) adhered to the membrane surface) and retain the filter performance. For example, Patent Document 2 proposes washing a membrane by the scheduled reverse permeation of permeate from the permeate side to maintain the filter performance. Patent Document 3 proposes performing filtration while washing a membrane surface with air supplied from below a filtration unit. Patent Document 4 proposes a method for chemical cleaning of a filtration unit to enable washing of a membrane surface that cannot be washed by the methods disclosed in Patent Documents 2 and 3. However, these treatment methods are for the washing of a membrane surface, and cannot control microbe concentrations. The chemical cleaning is particularly problematic, because it has adverse effects on the fermentation product, deteriorates the membrane and shortens the membrane lifetime, and requires a treatment of the waste liquid produced by the chemical cleaning.

Among the other techniques considered possible include: a technique that increases the washing effect by using high-temperature water as the backwashing water when conducting the backwashing using permeate (Patent Document 5); a separation membrane washing operation technique that involves a concentration step, a membrane separation step, and a warm-water washing step (Patent Document 6); and a separation membrane washing method that uses hot water and a hydrolase (Patent Document 7). However, it is difficult with these methods to control microbe proliferation, and the hydrolase is expensive and directly adds cost.

BACKGROUND ART DOCUMENT

PATENT DOCUMENT

Patent Document 1: WO2007/097260
Patent Document 2: JP-A-8-141375
Patent Document 3: JP-A-2001-38177
Patent Document 4: JP-A-2002-126470
Patent Document 5: JP-A-2008-289959
Patent Document 6: Japanese Patent No. 3577992
Patent Document 7: JP-A-2006-314883

SUMMARY OF THE INVENTION

PROBLEMS THAT THE INVENTION IS TO SOLVE

As described above, the conventional techniques do not address problems such as the complicated washing step, treatment of the waste liquid generated by the washing, and retention of filter performance and the control of the excessive microbe concentrations for the high-concentration culturing of microbes. Accordingly, there is a need for the development of a membrane filter operation method for retaining filter performance, and a method for controlling microbe concentrations.

It is an object of the present invention to provide a backwashing method capable of retaining filter performance for the high-concentration culturing of a microbe mixture, and at the same time controlling microbe concentrations in the production and collection of a product through fermentation using a separation membrane.

MEANS FOR SOLVING THE PROBLEMS

The present invention can solve the foregoing problems with the following configurations (1) to (5).

(1) A method for producing chemicals through continuous fermentation, the method comprising:

(a) generating chemicals by fermentation with a culture medium containing a fermentation feedstock and microbes or cultured cells at a temperature of 15 °C to 65 °C in s fermenter;
(b) filtering the culture medium through a separation membrane;
(c) collecting the chemical from the filtrate;
(d) retaining or refluxing unfiltered remains in the fermenter;
(e) adding fermentation feedstock to the culture medium in the fermenter; and
(f) washing the separation membrane with a washing liquid supplied from the permeate side of the separation membrane during step (a) in a state where the fermentation with the microbes or the cultured cells proceeds in the culture medium in the fermenter;

wherein the washing liquid is water which has a temperature at least 5 °C higher than the temperature of the culture medium in the fermenter in step (a) up to a maximum temperature of the washing water of 100 °C, and the concentration of the microbes in the fermenter is controlled by supplying the washing liquid.
(2) The method for producing chemicals through continuous fermentation according to (1), in which the washing liquid contains an oxidizing agent.
(3) The method for producing chemicals through continuous fermentation according to (2), in which the oxidizing agent contains at least agent selected from the group consisting of hypochlorite, chlorine dioxide, ozone, and hydrogen peroxide.
(4) The method for producing chemicals through continuous fermentation according to (1), in which the washing liquid contains a pH adjuster.
(5) The method for producing chemicals through continuous fermentation according to (1), in which the washing liquid contains the fermentation feedstock.

ADVANTAGE OF THE INVENTION

The present invention has advantages in the continuous fermentation operation performed to filter a fermentation culture medium with a separation membrane and to remove the chemical-containing permeate while continuously retaining the unfiltered remains in a fermentor. Specifically, the invention effectively enables washing of membrane foulants resulting from membrane filtration, and control of microbe concentrations in a fermentor. The invention can also greatly improve the fermentation production efficiency both stably and inexpensively, and can prevent generation of a waste washing liquid and a withdrawn culture medium, making it possible to reduce costs through reduction of the treatment cost. The invention can thus stably produce fermentation products at low cost in a wide range of fermentation industries.

BRIEF DESCRIPTION OF THE DRAWINGS

[Fig. 1] Fig. 1 is a schematic side view explaining an example of a membrane separation continuous fermentation device used in the present invention.
[Fig. 2] Fig. 2 is a diagram representing the method and the procedure of the backwashing performed with a membrane module in the present invention.
[Fig. 3] Fig. 3 is a diagram representing changes in microbe concentration over time in continuous fermentation filtration experiments according to Examples 1 to 3 and Comparative Examples 1 and 2.
[Fig. 4] Fig. 4 is a diagram representing changes in transmembrane pressure over time in continuous fermentation filtration experiments according to Examples 1 to 3 and Comparative Examples 1 and 2.
[Fig. 5] Fig. 5 is a diagram representing changes in microbe concentration over time in continuous fermentation filtration experiments according to Examples 4 and 5 and Comparative Example 3.
[Fig. 6] Fig. 6 is a diagram representing changes in transmembrane pressure over time in continuous fermentation filtration experiments according to Examples 4 and 5 and Comparative Example 3.

MODE FOR CARRYING OUT THE INVENTION

The present invention is a method for operating a continuous fermentation device in a continuous fermentation that uses a membrane module for filtration and in which the chemical-containing permeate is drawn out while continuously retaining the unfiltered remains in a fermentor. The method includes washing the membrane with high-temperature water supplied from the permeate side of the membrane module, and controlling the high-temperature water supply conditions according to the microbe concentration in the fermentor.

The separation membrane used for the membrane module of the present invention may be an organic membrane or an inorganic membrane, provided that it has chemical resistance. Examples thereof include polyvinylidene fluoride, polysulfone, polyethersulfone, polytetrafluoroethylene, polyethylene, polypropylene, and ceramic membranes.

The separation membrane used in the present invention is preferably a porous membrane having an average pore size of 0.01 $\mu$m or more and less than 1 $\mu$m. The separation membrane may have any shape, and may be, for example, a flat membrane, or a hollow fiber membrane.

The surface average pore size of the separation membrane is determined from the number average obtained by the measurement of randomly selected 20 pore diameters in a photograph of a membrane surface taken at 60,000x magnification with a scanning electron microscope. When the pores are not circular, the surface average pore size is determined by using a method in which the diameter of a circle (equivalent circle) having the same area as the pore, determined with a device such as an image processing device is used as the pore diameter.

In a preferred embodiment of the present invention, the separation membrane may be used to perform filtration over a transmembrane pressure of 0.1 to 20 kPa.

The membrane module of the present invention is not particularly limited, as long as it is made from materials having excellent heat resistance, and is shaped to allow high-temperature water to be injected toward the feed side from the permeate side of the module.

The feedstock for the fermentation of microbes and cultured cells used in the present invention is not particularly limited, as long as it can promote growth of the microbes to be fermentation-cultured or cultured cells to be fermentation-cultured, and can desirably produce chemicals as the intended products of the fermentation. Preferred examples of the fermentation feedstock include common liquid media that appropriately contain a carbon source, a nitrogen source, inorganic salts, and, as necessary, trace amounts of organic nutrients such as amino acids and vitamins. It is also possible to use, for example, wastewater or sewage, either directly or with the fermentation feedstock, provided that it is a liquid that partly contains materials that can promote growth of the microbes to be fermentation-cultured or cultured cells to be fermentation-cultured, and can desirably produce chemicals as the intended products of the fermentation.

Examples of the carbon source include sugars such as glucose, sucrose, fructose, galactose, and lactose; starches and starch hydrolysates containing such sugars; sweet potato syrup; sugar beet syrup; cane juice; extracts or concentrates of sugar beet syrup or cane juice; filtrates of sugar beet syrup or cane juice; raw sugars purified or crystallized from a syrup (high-test molasses), a sugar beet syrup, or a cane juice; white sugars purified or crystallized from a sugar beet or a cane juice; organic acids such as acetic acid and fumaric acid; alcohols such as ethanol; and glycerine. As used herein, sugars refer to the first polyalcohol oxidation products, and carbohydrates having one aldehyde group or one ketone group, classified as aldose in the case of the aldehyde group, or ketose in the case of the ketone group.

Examples of the nitrogen source include ammonium salts, urea, nitrates, and other organic nitrogen sources used as supplements. Other examples include oil meals, soybean hydrolysates, decomposed caseins, other amino acids, vitamins, corn steep liquor, yeasts or yeast extracts, meat extracts, peptides such as peptone, and various fermentative microbes and hydrolysates thereof.

Materials, for example, such as phosphates, magnesium salts, calcium salts, iron salts, and manganese salts may be appropriately used as the inorganic salts.

In the present invention, the microbe fermentation culture is brought to an appropriate pH and temperature according to the microbe species and the product productivity. Typically, the microbe fermentation culture is set to a pH of 4 to 8, and a temperature of 15 to 65°C. The pH of the fermentation culture medium is adjusted to a preset value of the foregoing range with materials such as inorganic acid or organic acid, alkaline substance, urea, calcium hydroxide, calcium carbonate, and ammonia.

The oxygen supply rate for the culture needs to be increased, this may be achieved by using various methods, including, for example, increasing the aeration amount, increasing the oxygen concentration by addition of oxygen to air, applying pressure to the fermentation culture medium, and increasing the agitation rate. On the other hand, if the oxygen supply rate needs to be lowered, this may be achieved by decreasing the aeration amount, or by supplying gases such as carbon dioxide gas, and oxygen-free gases such as nitrogen and argon with air.

In the present invention, the fermentation culture medium containing microbes or cultured cells is preferably drawn out in an amount appropriately adjusted according to the OD600 or MLSS (Mixed Liquor Suspended Solid) of the fermentation culture medium, so that the concentration of the microbes or cultured cells does not decrease and lower the productivity of the fermentation product.

Typically, the continuous culture procedure involving proliferation of fresh microbes capable of producing fermentation products is performed preferably in a single fermentor for the purpose of culture control. However, the number of fermentors is not limited, as long as the continuous fermentation culturing method that generates products through microbe proliferation is used. More than one fermentor may be used for reasons such as a small fermentor volume. In this case, high fermentation productivity can be obtained by performing continuous culturing with a plurality of fermentors connected to one another in parallel or in series.

Eukaryotic cells or prokaryotic cells are used as the microbes and cultured cells used in the present invention. Specifically, those commonly used in the fermentation industry are used, including, for example, fungi such as yeasts and filamentous fungi, microbes such as *Escherichia coli,* lactic acid bacteria, coryneform bacteria, and actinomycete, and animal cells and insect cells. The bacteria and cells may be those isolated from natural environment, or those with partially modified properties through mutation or genetic recombination.

The chemicals obtained by the production method of the present invention are substances produced by the microbes or cultured cells in the fermentation culture medium. Examples of the chemicals include substances mass produced in the fermentation industry, including, for example, alcohols, organic acids, amino acids, and nucleic acids. The present invention is also applicable to production of substances such as enzymes, antibiotics, and recombinant proteins. Examples of the alcohols include ethanol, 1,3-butanediol, 1,4-butanediol, and glycerol. Examples of the organic acids include acetic acid, lactic acid, pyruvic acid, succinic acid, malic acid, itaconic acid, and citric acid. Examples of the nucleic acids include inosine, guanosine, and cytidine.

The chemicals obtained by the production method of the present invention are preferably liquid products that contain at least one selected from chemical products, dairy products, drug products, and food or brewed products. Examples of the chemical products include substances, such organic acids, amino acids, and nucleic acids, that are applicable to chemical production through steps following the filtration by membrane separation. Examples of the dairy products include substances, such as low-fat milk, applicable to dairy production through steps following the filtration by membrane separation. Examples of the drug products include substances, such as enzymes, antibiotics, and recombinant proteins, applicable to drug production through steps following the filtration by membrane separation. Examples of the food include substances, such as lactic acid drinks, applicable to food production through steps following the filtration by membrane separation. Examples of the brewed products include substances, such as beer and shochu (distilled spirit), applicable to alcohol-containing drink production through steps following the filtration by membrane separation.

In the present invention, the transmembrane pressure for the filtration of the fermentation culture medium of microbes and cultured cells with the separation membrane of the membrane module is not limited, as long as the filtration proceeds under the conditions that do not easily allow the microbes, the cultured cells, and the medium components to clog the membrane. It is, however, important that the filtration is performed at a transmembrane pressure of 0.1 kPa to 20 kPa. The transmembrane pressure is preferably 0.1 kPa to 10 kPa, more preferably 0.1 kPa to 5 kPa. A transmembrane pressure outside these ranges may cause troubles in the continuous fermentation operation since the microbes and medium components easily clog the membrane, and lower the amounts of permeate.

The driving force of filtration may be provided by a liquid level difference (water head difference), or by the transmembrane pressure generated across the separation membrane with a cross-flow circulation pump. Alternatively, a suction pump may be installed on the permeate side of the separation membrane to provide a driving force of filtration. When a cross-flow circulation pump is used, the transmembrane pressure may be controlled with suction pressure. The transmembrane pressure also may be controlled by the pressure of the gas or the liquid that introduces pressure for the fermentation culture medium side. For the pressure control, the pressure difference between the pressure on the fermentation culture medium side and the pressure on the separation membrane permeate side may be used as the transmembrane pressure for the control of transmembrane pressure.

The present invention uses high-temperature water that has a membrane washing effect, and that can reduce the microbe concentration. The high-temperature water is set to a temperature 5°C or more higher than the culture medium temperature and less than 100°C, more preferably a temperature at least 10°C higher than the culture temperature and less than 100°C. Temperatures below the culture medium temperature make it difficult to control microbe concentrations in the continuous fermentation. On the other hand, if the temperature exceeds 150°C, a very high pressure needs to be applied to maintain the high-temperature water in the liquid state. Temperatures in these ranges are therefore not practical. If the backwashing water has a higher temperature than in the foregoing conditions it raises the possibility of rapidly killing large numbers of microbes in a manner that depends on the microbes characteristics. It is therefore desirable to measure the temperature-dependent kill rate of the microbes in advance.

As used herein, backwashing refers to a method in which liquid is sent from the permeate side to the feed side of the separation membrane, thereby removing the foulants from the membrane surface.

The high-temperature water used as the backwashing liquid is not particularly limited, as long as it is not contaminated by microbes, and does not contain substances that have the risk of membrane fouling.

The high-temperature water may be supplied using a constant-temperature unit, or using a heater provided for a water supply pipe. It is preferable to check the temperature of the high-temperature water with a thermometer, and to control the temperature within ± 1°C of the preset temperature.

The supply rate of the high-temperature water is desirably 1 to 3 times the permeation flux of the membrane, or may be set to a more appropriate rate taking into account such factors as the microbe concentration, and the membrane washing effect.

The high-temperature water of the present invention may contain washing agents, specifically, oxidizing agents such as sodium hypochlorite, chlorine dioxide, ozone and hydrogen peroxide, and pH adjusters such as sodium hydroxide, calcium hydroxide, hydrochloric acid and sulfuric acid, provided that such addition does not inhibit the effects of the present invention. Further, high-temperature water containing a fermentation feedstock may be used in order to prevent the concentration of the fermentation feedstock in the culture medium from being lowered.

In the present invention, a liquid is sent from the permeate side to the feed side of the membrane module while maintaining the temperature of the backwashing liquid high, thereby washing the membrane and controlling the microbe concentration. When the high-temperature backwashing liquid is water, the high-temperature water makes it easier to detach the adhered materials from the separation membrane, and the proliferation of the microbes is halted by contacting with the high-temperature water.

It is considered that the high-temperature water helps detachment of the adhered substances from the separation membrane, and, when the substance adhered to the membrane is a carbohydrate-derived substance, the high-temperature water creates an environment where the adhered substance can easily be dissolved. The substance adhered to the membrane can thus dissolve into the high-temperature water. On the other hand, when the substance adhered to the membrane is a protein-derived substance, the high-temperature water denatures the protein and changes the characteristics of the membrane adhesion, making it easier to detach the substance from the membrane.

When the high-temperature water is one that contains the washing agent, detachment from the membrane can be promoted by the components derived from the substances adhered to the membrane. For example, sodium hypochlorite as an example of the washing agent is a strong oxidizing agent, and, when it is used as a high-temperature oxidizing agent for the backwashing, the oxidation effect of the carbohydrate-derived substances adhered to the membrane is promoted, thereby obtaining a higher membrane washing effect than that provided by the high-temperature water alone. On the other hand, sodium hydroxide and calcium hydroxide as examples of the washing agent are strong alkaline agents, and, when they are used as a high-temperature alkali for the backwashing, the denature effect of the protein-derived substance adhered to the membrane is promoted, thereby obtaining a higher membrane washing effect than that provided by the high-temperature water alone. Note that, although these strong oxidizing agents and the strong alkaline agents used at high temperatures allow for the control of microbe proliferation, the addition of the high-temperature water may pose the risk of deactivating the microbes or rapidly increasing the kill rate of the microbes when the concentration exceeds a certain range. It is therefore important that the washing agent be used in a concentration range that allows for the membrane washing and the control of microbe concentrations.

In present invention, using the washing agent in the range that does not inhibit the effects of the present invention means using preferably, for example, a washing liquid having an effective chlorine concentration of 1 to 5,000 ppm in the case of sodium hypochlorite, and preferably, for example, a washing liquid having a pH of 10 to 13 in the case of sodium hydroxide and calcium hydroxide. Concentrations above this range have possible damage to the separation membrane or possible adverse effects on the microbes. Concentrations below this range raise concerns over the lowering of the membrane washing effect.

The backwashing cycle of the high-temperature water used as the backwashing liquid may be determined from the transmembrane pressure and from changes in transmembrane pressure. The backwashing cycle is in a range from 0.1 to 12 cycles/hour, more preferably 3 to 6 cycles/hour. A backwashing cycle above these ranges has the possible risk of deactivating the microbes upon adding the high-temperature water or rapidly increasing the microbe kill rate. Below these ranges, the washing effect and the microbe control effect may not be obtained sufficiently.

The backwashing time for the high-temperature water used as the backwashing liquid may be determined from the backwashing cycle, the transmembrane pressure, and changes in the transmembrane pressure. The backwashing time is in a range from 5 to 300 seconds/cycle, more preferably 30 to 180 seconds/cycle. A backwashing time above these ranges has the possible risk of deactivating the microbes upon adding the high-temperature water or rapidly increasing the microbe kill rate. Below these ranges, the washing effect and the microbe control effect may not be obtained sufficiently.

The pipes and valves used for a constant-temperature tank, a high-temperature water backwash pump, and between a constant-temperature tank and the module may be selected from those having excellent heat resistance. The high-temperature water may be injected either manually, or, more desirably, automatically by controlling a filter pump and a filter valve, and a high-temperature water backwash pump and a high-temperature water backwash valve by using a filtration and backwash control unit, using a timer or the like.

The present invention requires monitoring microbe concentrations for the control of the microbe concentration in the fermentor. Microbe concentrations may be measured by collecting a sample. However, it is more desirable to continuously monitor the changes in microbe concentration by using a microbe concentration sensor, for example, such as an MLSS measurement device, installed in the fermentor.

The continuous fermentation device used in the present invention is described below with reference to the accompanying drawings.

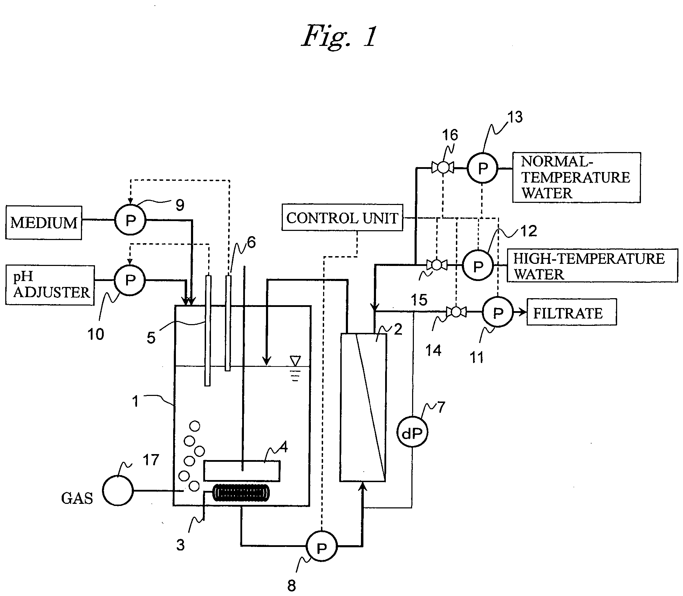

Fig. 1 is a schematic side view explaining an example of a continuous fermentation device used in the method of supplying high-temperature water of the present invention. The continuous fermentation device depicted in Fig. 1 is a representative example in which the separation membrane module is installed outside of the fermentor. In Fig. 1, the continuous fermentation device is basically configured from a fermentor 1, a separation membrane module 2, and a high-temperature water supply unit. Large numbers of hollow fiber membranes are incorporated in the separation membrane module 2. A normal-temperature water supply unit is configured from a normal-temperature water backwash pump 13 and a normal-temperature water backwash valve 16. The high-temperature water supply unit is configured from a high-temperature water backwash pump 12 and a high-temperature water backwash valve 15. A filter unit is configured from a filter pump 11 and a filter valve 14. The separation membrane module and the high-temperature water supply unit will be described later in detail. The separation membrane module 2 is connected to the fermentor 1 via a circulation pump 8.

In Fig. 1, a level sensor-control unit 6 and a medium supply pump 9 can charge a medium into the fermentor 1 to control the liquid level in the fermentor, and, as required, an agitator 4 can agitate the culture medium in the fermentor 1. The gas supply unit 17 can supply the necessary gas, as required. Here, the supplied gas may be resupplied by the gas supply unit 17 after being collected and recycled. Further, as required, a pH sensor-control unit 5 and a pH adjuster supply pump 10 can adjust the pH of the fermentation culture medium to produce fermentation products at high productivity.

In the device, the circulation pump 8 circulates the fermentation culture medium between the fermentor 1 and the separation membrane module 2. The fermentation culture medium containing the fermentation product can be drawn out from the device after being filtered and separated into the microbes and the fermentation product with the separation membrane module 2. The separated microbes remain in the device, and can thus be maintained at high concentrations therein, making it possible to produce fermentation products at high productivity. The filtration and separation by the separation membrane module 2 may be performed by using the pressure of the circulation pump 8, without requiring a special power. However, a filter pump 11 may be optionally provided, and a differential pressure sensor-control unit 7 may be used to appropriately adjust the filtrate amount. As required, a temperature control unit 3 may be used to maintain the temperature of the fermentor 1 constant, and maintain high microbe concentrations.

The high-temperature water supply unit used in the method of supplying high-temperature water in the present invention is configured from the high-temperature water backwash pump 12 and the high-temperature water backwash valve 15. The high-temperature water may be introduced while monitoring microbe concentrations.

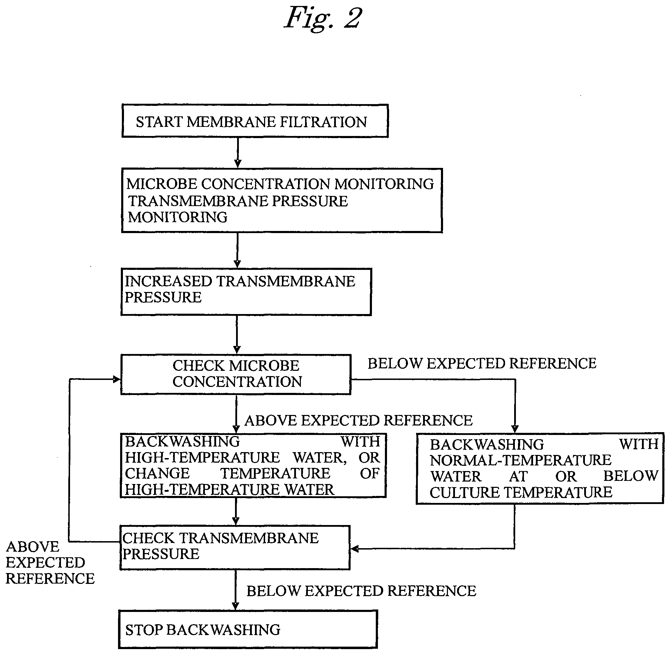

The control in the operation method of the present invention is performed according to the procedure represented in Fig. 2.

The procedure starts with the membrane filtration of the culture medium, and changes in microbe concentration and transmembrane pressure are monitored. The microbe concentration is checked when the transmembrane pressure increased as a result of membrane foulants in the membrane filtration, and backwashing is performed with the high-temperature water when the concentration exceeds the expected microbe concentration.

The microbe concentration is checked upon an increase of the transmembrane pressure, and, when the concentration is below the expected microbe concentration, backwashing is performed with the normal-temperature water having a temperature no greater than the culture temperature.

Following the membrane washing with the high-temperature water or with the normal-temperature water of a temperature no greater than the culture temperature, the transmembrane pressure is checked, and the washing effect is confirmed. When the transmembrane pressure exceeds the expected reference transmembrane pressure, the microbe concentration is checked again. The backwashing may be stopped when the transmembrane pressure is below the expected reference transmembrane pressure. The expected reference transmembrane pressure may be determined by the properties of the separation membrane, and the properties of the separation membrane module.

The backwashing performed with the high-temperature water in the manner described above makes it possible to wash foulants on the separation membrane without generating a waste liquid, and to control the concentration of the microbes proliferated in excess. The high-temperature water supply method that enables the retention of the filter performance and the control of microbe concentrations can be performed to improve the filter performance of the membrane module and to enable a stable filtration operation over extended time periods in the filtration of a microbe culture medium.

EXAMPLES

Example 1

First, the membrane module was fabricated. The hollow fiber membrane used for the fabrication of the membrane module was prepared by disassembling the Toray pressure polyvinylidene fluoride hollow fiber membrane module HFS1020, and cutting out only the portion of the polyvinylidene fluoride hollow fiber membrane not attached and fixed to the module. A molded product of polycarbonate resin was used as a separation membrane module member. The membrane module had a volume of 0.06 L, and an effective filter area of 200 cm$^2$. The porous hollow fiber membrane and the membrane module so fabricated were used to perform a continuous fermentation. The operation conditions used in Example 1 are as follows, unless otherwise stated.

Operation Conditions

- Fermentor volume: 2.0 L
- Fermentor effective volume: 1.5 L
- Separation membrane used: Polyvinylidene fluoride hollow fiber membrane (60 fibers)
- Adjusted temperature: 37°C
- Aeration amount through fermentor: 0.2 L/min
- Fermentor agitation rate: 600 rpm
- Adjusted pH: pH 6 with 3N NaOH
- Lactic acid fermentation medium supply rate: Variably controlled in a 15 to 300 mL/hr range
- Liquid circulation amount by culture medium circulator: 3.5 L/min
- Control of membrane filter flow amount: Flow amount was controlled with a suction pump
- Sterilization: Fermentor and medium, including membrane module were all sterilized under high-temperature steam with an autoclave for 20 minutes at 121°C.

*Sprolactobacillus laevolacticus* JCM2513 (SL strain) was used as microbes, and the lactic acid fermentation medium of the composition presented in Table 1 was used as the medium. The concentration of the product lactic acid was evaluated under the following conditions, using the HPLC below.

Table 1
Lactic acid fermentation medium

| Components | Concentration |
|---|---|
| Glucose | 100 g/L |
| Yeast Nitrogen base w/o amino acid (Difco) | 6.7 g/L |
| Standard 19 amino acids excluding leucine | 152 mg/L |
| Leucine | 760 mg/L |
| Inositol | 152 mg/L |
| p-Aminobenzoic acid | 16 mg/L |
| Adenine | 40 mg/L |
| Uracil | 152 mg/L |

- Column: Shim-Pack SPR-H (Shimadzu)

- Mobile phase: 5 mM p-toluene sulfonic acid (0.8 mL/min)

- Reaction phase: 5 mM p-toluene sulfonic acid, 20 mM bistris, 0.1 mM EDTA.2Na (0.8 mL/min)

- Detection method: Electrical conductivity

- Column temperature: 45°C

The optical purity of the lactic acid was analyzed under the following conditions.

- Column: TSK-gel Enantio L1 (Tosoh)

- Mobile phase: 1 mM copper sulfate aqueous solution

- Flow amount: 1.0 mL/min

- Detection method: UV 254 nm

- Temperature: 30°C

The optical purity of the L-lactic acid is calculated according to the following equation (i).

$$\text{Optical purity (\%)} = 100 \times (L - D)/(D + L) \qquad (i)$$

The optical purity of the D-lactic acid is calculated according to the following equation (ii).

$$\text{Optical purity (\%)} = 100 \times (D - L)/(D + L) \qquad (ii)$$

In the equations, L represents the concentration of the L-lactic acid, and D represents the concentration of the D-lactic acid.

For culturing, the SL strain was shaken and cultured overnight in a test tube containing a 5-mL lactic acid fermentation medium (first preculture). The resulting culture medium was inoculated into a fresh lactic acid fermentation medium (100 mL), and shaken and cultured for 24 hours at 37°C in a 500 mL-volume Sakaguchi flask (second preculture). The second preculture medium was inoculated into a medium in a 1.5-L fermentor of the continuous fermentation device shown in Fig. 1, and agitated with the agitator 4. After adjusting the aeration amount through the fermentor 1, and the temperature and pH, the culture was grown for 50 hours without operating the circulation pump 8 (final preculture). Immediately after the final preculture, the circulation pump 8 was operated, and the lactic acid fermentation medium was continuously supplied under the final-preculture operating conditions while additionally controlling the amount of the permeate through the membrane so that the amount of the culture medium in the membrane separation continuous fermentation device becomes 1.5 L. The continuous culture produced D-lactic acid through continuous fermentation. In the continuous fermentation test, the amount of the permeate through the membrane was controlled by measuring the amount of the filtrate leaving the filter pump 11, and by varying the filtrate amount through the membrane under controlled conditions. The product D-lactic acid in the membrane-filtered culture medium was appropriately measured for concentration and optical purity.

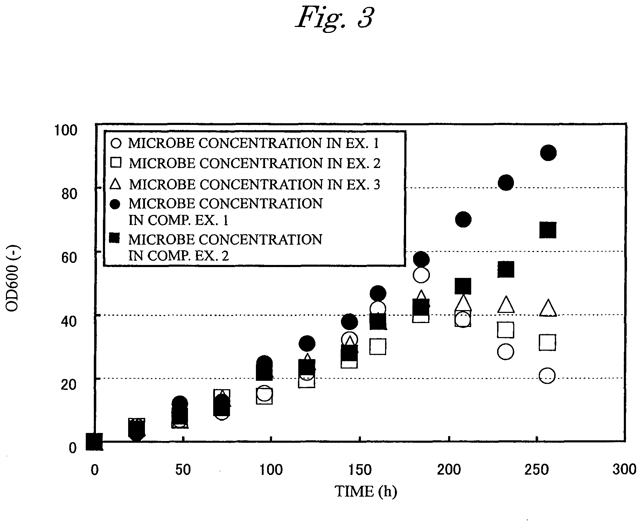

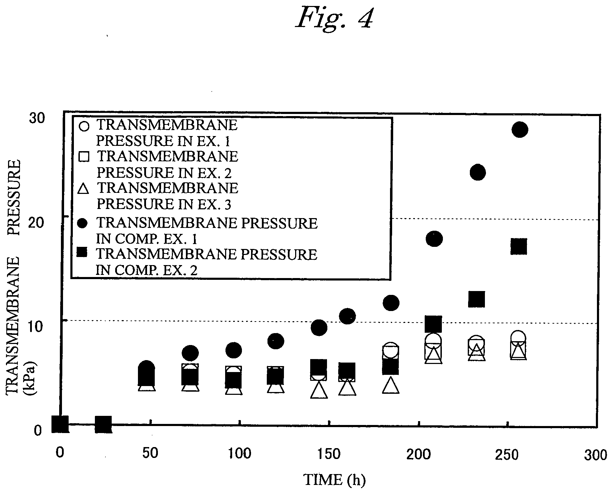

The continuous fermentation filtration operation was performed for 250 hours. The membrane filtration operation was performed with the filter pump 11. The filtration flow amount was zero from hour 0 to hour 50, 100 mL/h from hour 50 to hour 200, and 135 mL/h from hour 200 to hour 250. The filtration was performed for 9 minutes in a repeated cycle, each time followed by a 1-min pause. The backwashing was continued from hour 50 to hour 250 of the filtration, and then for 1 min at a flow amount of 600 mL/h after 9 minutes of filtration. The high-temperature water used for the backwashing was prepared by charging distilled water in the constant-temperature unit and maintaining the water temperature constant. The high-temperature water used for the backwashing was maintained at 90°C according to the temperature setting of the constant-temperature unit, and was used for the continuous fermentation from hour 200 to hour 250. Normal temperature water was used for the backwashing in other times when the high-temperature water was not used. Since the microbe concentration in the fermentor tends to lower the filter performance when increased, the operation was performed at a microbe concentration expected to appropriately maintain the filtration rate and productivity. The transmembrane pressure was measured once a day with a differential pressure meter, whereas the microbe concentration was measured once a day by taking an OD600. For the OD600 measurement, a sample was first collected from the fermentor, and diluted with a physiological saline to make the sample OD600 1 or less. The absorbance at 600-nm wavelength was then measured with a spectrophotometer (UV-2450; Shimadzu Corporation). The measured value was multiplied by the factor used for the dilution with the physiological saline, and the product of the calculation was obtained as the sample OD600. The results of the experiments are presented in Figs. 3 and 4. As a result, it was possible to lower the microbe concentration to an OD600 of about 20, and stably maintain the transmembrane pressure. In other words, effective membrane washing was possible under controlled microbe concentrations.

Example 2

A continuous fermentation test for D-lactic acid was conducted in the same manner as in Example 1, except that the high-temperature water for backwashing was used for the continuous fermentation from hour 200 to hour 250 after being maintained at 60°C according to the temperature setting of the constant-temperature unit. The results are presented in Figs. 3 and 4. As a result, it was possible to lower the microbe concentration to an OD600 of about 30, and stably maintain the transmembrane pressure. In other words, effective membrane washing was possible under controlled microbe concentrations.

Example 3

A continuous fermentation test for D-lactic acid was conducted in the same manner as in Example 1, except that the high-temperature water for backwashing was used for the continuous fermentation from hour 200 to hour 250 after being maintained at 45°C according to the temperature setting of the constant-temperature unit. The results are presented in Figs. 3 and 4. As a result, it was possible to lower the microbe concentration to an OD600 of about 40, and stably maintain the transmembrane pressure. In other words, effective membrane washing was possible under controlled microbe concentrations.

Example 4

A continuous fermentation was performed for microbes that produce pyruvic acid, using the same membrane module used in Example 1. Specifically, the P120-5a strain (FERM P-16745) of the yeast *Torulopsis glabrata* was used as the pyruvic acid-producing microbes.

A continuous fermentation test was conducted with the continuous fermentation device of Fig. 1. The pyruvic acid fermentation medium of the composition presented in Table 2 was used as the fermentation medium. The pyruvic acid fermentation medium was used after being sterilized under high-pressure steam for 20 min at 121°C. The operation was performed under the following conditions, unless otherwise stated.

Table 2

| Pyruvic acid fermentation medium | |
| --- | --- |
| Components | Concentration |
| Glucose | 100 g/L |
| Ammonium sulfate | 5 g/L |
| Potassium dihydrogenphosphate | 1 g/L |
| Magnesium sulfate heptahydrate | 0.5 g/L |
| Soy hydrolysate | 2 g/L |
| Nicotinic acid | 8 mg/L |
| Pyridoxine-hydrochloride | 1 mg/L |
| Biotin | 0.05 mg/L |
| Thiamine-hydrochloride | 0.05 mg/L |
| *pH = 5.5 | |

Operation Conditions

- Fermentor volume: 2.0 L
- Fermentor effective volume: 1.5 L
- Separation membrane: Polyvinylidene fluoride hollow fiber membrane (60 fibers)
- Adjusted temperature: 30°C
- Aeration amount through fermentor: 1.5 L/min
- Fermentor agitation rate: 800 rpm
- Adjusted pH: pH 5.5 with 4N NaOH
- Pyruvic acid fermentation medium supply rate: Variably controlled in a 15 to 300 mL/hr range
- Liquid circulation amount with culture medium circulator: 3.5 L/min
- Control of membrane filtration flow amount: Flow amount was controlled with a suction pump
- Sterilization: Fermentor and medium, including membrane module were all sterilized under high-temperature steam with an autoclave for 20 minutes at 121°C.

The pyruvic acid concentration was evaluated by HPLC measurement performed under the following conditions.

- Column: Shim-Pack SPR-H (Shimadzu)
- Mobile phase: 5 mM p-toluene sulfonic acid (flow amount 0.8 mL/min)
- Reaction liquid: 5 mM p-toluene sulfonic acid, 20 mM bistris, 0.1 mM EDTA.2Na (flow amount 0.8 mL/min)
- Detection method: Electrical conductivity
- Temperature: 45°C

The Glucose Test Wako C® (Wako Pure Chemical Industries, Ltd.) was used for the measurement of glucose concentration.

For culturing, the P120-5a strain was shaken and cultured overnight in a test tube containing a 5-mL pyruvic acid fermentation medium (first preculture). The resulting culture medium was inoculated into a fresh pyruvic acid fermentation medium (100 mL), and shaken and cultured for 24 hours at 30°C in a 500 mL-volume Sakaguchi flask (second preculture). The second preculture medium was inoculated into a 1.5-L pyruvic acid fermentation medium in the continuous fermentation device shown in Fig. 1, and the fermentor 1 was agitated with the agitator 4. After adjusting the aeration amount through the fermentor 1, and the temperature and pH, the culture was grown for 24 hours without operating the circulation pump 8 (final preculture). Immediately after the final preculture, the circulation pump 8 was operated, and the pyruvic acid fermentation medium was continuously supplied under the final-preculture operating conditions while additionally controlling the amount of the permeate through the membrane so that the amount of the culture medium in the continuous fermentation device becomes 1.5 L. The continuous culture produced pyruvic acid through continuous fermentation. In the continuous fermentation test, the amount of the permeate through the membrane was controlled by measuring the amount of the filtrate leaving the filter pump 11, and by varying the filtrate amount through the membrane under controlled conditions. The concentrations of the product pyruvic acid and the residual glucose in the membrane-filtered culture medium were appropriately measured.

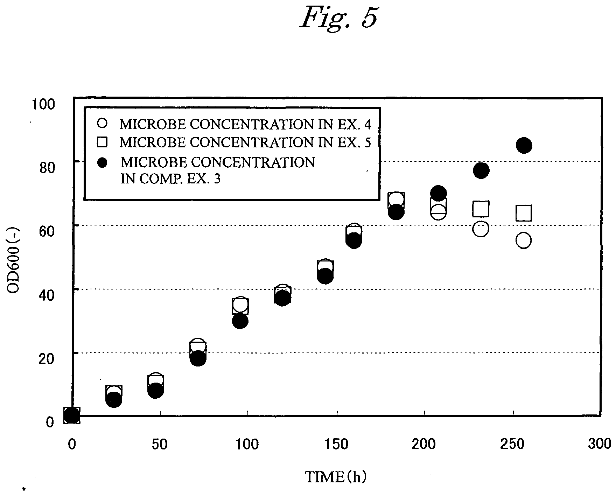

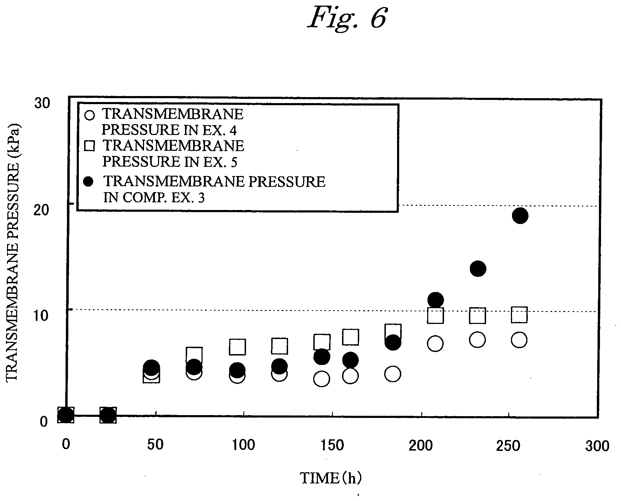

The continuous fermentation filtration operation was performed for 250 hours. The membrane filtration operation was performed with the filter pump 11. The filtration flow amount was zero from hour 0 to hour 50, 100 mL/h from hour 50 to hour 200, and 135 mL/h from hour 200 to hour 250. The filtration was performed for 9 minutes in a repeated cycle, each time followed by a 1-min pause. The backwashing was continued from hour 50 to hour 250 of the filtration, and then for 1 min at a flow amount of 600 mL/h after 9 minutes of filtration. The high-temperature water used for the backwashing was prepared by charging distilled water in the constant-temperature unit and maintaining the water temperature constant. The high-temperature water used for the backwashing was maintained at 50°C according to the temperature setting of the constant-temperature unit, and was used for the continuous fermentation from hour 200 to hour 250. Normal temperature water was used for the backwashing in other times when the high-temperature water was not used. Since the microbe concentration in the fermentor tends to lower the filter performance when increased, the operation was performed at a microbe concentration expected to appropriately maintain the filtration rate and productivity. The transmembrane pressure was measured once a day with a differential pressure meter, whereas the microbe concentration was measured once a day by taking an OD600. For the OD600 measurement, a sample was first collected from the fermentor, and diluted with a physiological saline to make the sample OD600 1 or less. The absorbance at 600-nm wavelength was then measured with a spectrophotometer (UV-2450; Shimadzu Corporation). The measured value was multiplied by the factor used for the dilution with the physiological saline, and the product of the calculation was obtained as the sample OD600. The results of the experiments are presented in Figs. 5 and 6. As a result, it was possible to lower the microbe concentration to an OD600 of about 55, and stably maintain the transmembrane pressure. In other words, effective membrane washing was possible under controlled microbe concentrations.

Example 5

A continuous fermentation test for pyruvic acid was conducted in the same manner as in Example 4, except that the high-temperature water for backwashing was used for the continuous fermentation from hour 200 to hour 250 after being maintained at 40°C according to the temperature setting of the constant-temperature unit. The results are presented in Figs. 5 and 6. As a result, it was possible to lower the microbe concentration to an OD600 of about 65, and stably maintain the transmembrane pressure. In other words, effective membrane washing was possible under controlled microbe concentrations.

Comparative Example 1

A continuous fermentation test for D-lactic acid was conducted in the same manner as in Example 1, without the backwashing with the high-temperature water. However, in order to take into consideration the possible dilution of the culture medium with the high-temperature water used for the backwashing, water was introduced through a medium inlet in the same amount as that used in Example 1 for the high-temperature water in the backwashing. The results are presented in Figs. 3 and 4. The OD600 increased to about 90 after 250 hours of continuous fermentation, and it was not possible to control the microbe concentration. Further, the transmembrane pressure increased, and a stable continuous fermentation filtration operation was not possible.

Comparative Example 2

A continuous fermentation test for D-lactic acid was conducted in the same manner as in Example 1, using the high-temperature water for the backwashing after setting the backwashing water temperature to 35°C. The results are presented in Figs. 3 and 4. The OD600 increased to about 70 after 250 hours of continuous fermentation, and it was not possible to control the microbe concentration. Further, the transmembrane pressure increased, and a stable continuous fermentation filtration operation was not possible.

Comparative Example 3

A continuous fermentation test for pyruvic acid was conducted in the same manner as in Example 4, except that the continuous fermentation was performed from hour 200 to hour 250 with the high-temperature water for the backwashing after setting the water temperature to 25°C according to the temperature setting of the constant-temperature unit. The results are presented in Figs. 5 and 6. The OD600 increased to about 85 after 250 hours of continuous fermentation, and it was not possible to control the microbe concentration. Further, the transmembrane pressure increased, and a stable continuous fermentation filtration operation was not possible.

INDUSTRIAL APPLICABILITY

The present invention provides a simple operation method that effectively enables washing of membrane foulants resulting from membrane filtration, and controlling microbe concentrations in a fermentor. The invention can also greatly improve the fermentation production efficiency both stably and inexpensively, and can reduce costs through reduction of the treatment costs resulting from a waste washing liquid and a withdrawn culture medium. The invention can thus stably produce fermentation products at low cost in a wide range of fermentation industries.

DESCRIPTION OF REFERENCE NUMERALS AND SIGNS

| 1  | Fermentor |
| 2  | Separation membrane module |
| 3  | Temperature control unit |
| 4  | Agitator |
| 5  | pH Sensor-control unit |
| 6  | Level sensor-control unit |
| 7  | Differential pressure sensor-control unit |
| 8  | Circulation pump |
| 9  | Medium supply pump |
| 10 | pH Adjuster supply pump |
| 11 | Filter pump |
| 12 | High-temperature water backwash pump |
| 13 | Normal-temperature water backwash pump |
| 14 | Filter valve |
| 15 | High-temperature water backwash valve |
| 16 | Normal-temperature water backwash valve |
| 17 | Gas supply unit |

**Claims**

1.  A method for producing chemicals through continuous fermentation, the method comprising:

    (a) generating chemicals by fermentation with a culture medium containing a fermentation feedstock and microbes or cultured cells at a temperature of 15 °C to 65 °C in a fermenter;
    (b) filtering the culture medium through a separation membrane;
    (c) collecting the chemical from the filtrate;
    (d) retaining or refluxing unfiltered remains in the fermenter,
    (e) adding a fermentation feedstock to the culture medium in the fermenter, and
    (f) washing the separation membrane with a washing liquid supplied from the permeate side of the separation membrane during step (a) in a state where the fermentation with the microbes or the cultured cells proceeds in the culture medium in the fermenter;

    wherein the washing liquid is water which has a temperature at least 5 °C higher than the temperature of the culture medium in the fermenter of step (a) up to a maximum temperature of the washing water of 100 °C, and the concentration of the microbes in the fermenter is controlled by supplying the washing liquid.

2.  The method for producing chemicals through continuous fermentation according to claim 1, wherein the washing liquid contains an oxidizing agent.

3.  The method for producing chemicals through continuous fermentation according to claim 2, wherein the oxidizing agent contains at least one oxidizing agent selected from the group consisting of hypochlorite, chlorine dioxide, ozone, and hydrogen peroxide.

4.  The method for producing chemicals through continuous fermentation according to claim 1, wherein the washing liquid contains a pH adjuster.

**5.** The method for producing chemicals through continuous fermentation according to claim 1, wherein the washing liquid contains the fermentation feedstock.

**Patentansprüche**

**1.** Verfahren zur Herstellung von Chemikalien durch kontinuierliche Fermentation, wobei das Verfahren umfasst:

(a) Erzeugen von Chemikalien durch Fermentation mit einem Kulturmedium, das einen Fermentationsrohstoff und Mikroben oder kultivierte Zellen enthält, bei einer Temperatur von 15 °C bis 65 °C in einem Fermenter;
(b) Filtrieren des Kulturmediums durch eine Trennmembran;
(c) Sammeln der Chemikalie aus dem Filtrat;
(d) Zurückhalten oder Rückfließen von ungefilterten Resten im Fermenter,
(e) Hinzufügen eines Fermentationsrohstoffs zu dem Kulturmedium im Fermenter, und
(f) Waschen der Trennmembran mit einer Waschflüssigkeit, die von der Permeatseite der Trennmembran während Schritt (a) in einem Zustand zugeführt wird, in dem die Fermentation mit den Mikroben oder den kultivierten Zellen im Kulturmedium im Fermenter abläuft;

wobei die Waschflüssigkeit Wasser ist, dessen Temperatur mindestens 5 °C höher ist als die Temperatur des Kulturmediums im Fermenter von Schritt (a) bis zu einer Maximaltemperatur des Waschwassers von 100 °C, und die Konzentration der Mikroben im Fermenter durch Zufuhr der Waschflüssigkeit kontrolliert wird.

**2.** Verfahren zur Herstellung von Chemikalien durch kontinuierliche Fermentation nach Anspruch 1, wobei die Waschflüssigkeit ein Oxidationsmittel enthält.

**3.** Verfahren zur Herstellung von Chemikalien durch kontinuierliche Fermentation nach Anspruch 2, wobei das Oxidationsmittel mindestens ein Oxidationsmittel enthält, ausgewählt aus der Gruppe bestehend aus Hypochlorit, Chlordioxid, Ozon, und Wasserstoffperoxid.

**4.** Verfahren zur Herstellung von Chemikalien durch kontinuierliche Fermentation nach Anspruch 1, wobei die Waschflüssigkeit ein pH-Einstellmittel enthält.

**5.** Verfahren zur Herstellung von Chemikalien durch kontinuierliche Fermentation nach Anspruch 1, wobei die Waschflüssigkeit den Fermentationsrohstoff enthält.

**Revendications**

**1.** Procédé de production de produits chimiques par fermentation continue, le procédé comprenant le fait :

(a) de générer des produits chimiques par fermentation avec un milieu de culture contenant une charge de fermentation et des microbes ou des cellules mises en culture à une température comprise entre 15°C et 65°C dans un fermenteur ;
(b) de filtrer le milieu de culture à travers une membrane de séparation ;
(c) de récupérer le produit chimique depuis le filtrat ;
(d) de conserver ou porter à reflux les restes non filtrés dans le fermenteur,
(e) d'ajouter une charge de fermentation au milieu de culture dans le fermenteur, et
(f) de laver la membrane de séparation avec un liquide de lavage fourni du côté du perméat de la membrane de séparation au cours de l'étape (a) dans un état où la fermentation avec les microbes ou les cellules mises en culture se déroule dans le milieu de culture dans le fermenteur ;

dans lequel le liquide de lavage est de l'eau qui a une température supérieure d'au moins 5°C à la température du milieu de culture dans le fermenteur à l'étape (a) jusqu'à une température maximale de l'eau de lavage de 100°C, et la concentration des microbes dans le fermenteur est régulée par alimentation en liquide de lavage.

**2.** Procédé de production de produits chimiques par fermentation continue selon la revendication 1, dans lequel le liquide de lavage contient un agent oxydant.

**3.** Procédé de production de produits chimiques par fermentation continue selon la revendication 2, dans lequel l'agent oxydant contient au moins un agent oxydant choisi dans le groupe constitué par l'hypochlorite, le dioxyde de chlore, l'ozone et le peroxyde d'hydrogène.

**4.** Procédé de production de produits chimiques par fermentation continue selon la revendication 1, dans lequel le liquide de lavage contient un régulateur de pH.

**5.** Procédé de production de produits chimiques par fermentation continue selon la revendication 1, dans lequel le liquide de lavage contient la charge de fermentation.

*Fig. 1*

# Fig. 2

```
┌─────────────────────────────────────────┐
│        START MEMBRANE FILTRATION         │
└─────────────────────────────────────────┘
                    │
                    ▼
┌─────────────────────────────────────────┐
│ MICROBE CONCENTRATION MONITORING         │
│ TRANSMEMBRANE PRESSURE                    │
│ MONITORING                                │
└─────────────────────────────────────────┘
                    │
                    ▼
┌─────────────────────────────────────────┐
│ INCREASED TRANSMEMBRANE                   │
│ PRESSURE                                  │
└─────────────────────────────────────────┘
                    │
                    ▼
┌──────────────────────────┐   BELOW EXPECTED REFERENCE
│     CHECK MICROBE         │─────────────────────────────┐
│     CONCENTRATION         │                             │
└──────────────────────────┘                             │
         │  ABOVE EXPECTED REFERENCE                      │
         ▼                                                ▼
┌──────────────────────────────┐    ┌──────────────────────────────┐
│ BACKWASHING          WITH    │    │ BACKWASHING          WITH     │
│ HIGH-TEMPERATURE WATER, OR   │    │ NORMAL-TEMPERATURE            │
│ CHANGE    TEMPERATURE    OF  │    │ WATER   AT   OR   BELOW       │
│ HIGH-TEMPERATURE WATER       │    │ CULTURE TEMPERATURE           │
└──────────────────────────────┘    └──────────────────────────────┘
         │                                          │
         ▼                                          │
┌──────────────────────────┐                        │
│ CHECK TRANSMEMBRANE      │◄───────────────────────┘
│ PRESSURE                 │
└──────────────────────────┘
         │ BELOW EXPECTED REFERENCE
         ▼
┌──────────────────────────┐
│ STOP BACKWASHING         │
└──────────────────────────┘
```

ABOVE EXPECTED REFERENCE

*Fig. 3*

# *Fig. 4*

*Fig. 5*

*Fig. 6*

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2007097260 A
- JP 8141375 A
- JP 2001038177 A
- JP 2002126470 A
- JP 2008289959 A
- JP 3577992 B
- JP 2006314883 A